# EUROPEAN PATENT APPLICATION

(11) **EP 3 907 223 A1**
(43) Date of publication of application: **10.11.2021**
(21) Application number: 20854609.3
(22) Date of filing: 24.04.2020
(51) Int. Cl.: C07D 413/04, C07D 498/14

(54) **METHOD FOR PREPARING DRUG TOXIN PNU-159682 OF ANTIBODY DRUG CONJUGATE, AND INTERMEDIATE THEREIN**

(30) Priority: 22.08.2019 CN 201910779777
(71) Applicant: Levena Biopharma Co., Ltd., SIP Suzhou Jiangsu 215123 (CN)
(72) Inventor: KONG, Lingpei, Suzhou, Jiangsu 215123 (CN); JIA, Zhongquan, Suzhou, Jiangsu 215123 (CN); ZHANG, Binbin, Suzhou, Jiangsu 215123 (CN); CHEN, Yu, Suzhou, Jiangsu 215123 (CN); XIA, Deyin, Suzhou, Jiangsu 215123 (CN); GUI, Dong, Suzhou, Jiangsu 215123 (CN); LI, Haihong, Suzhou, Jiangsu 215123 (CN); GUO, Maojun, Suzhou, Jiangsu 215123 (CN); LI, Hui, Suzhou, Jiangsu 215123 (CN)
(74) Representative: Sach, Greg Robert
(86) International application number: PCT/CN2020/086631
(87) International publication number: WO 2021/031599

(57) **Abstract**

The disclosure provides a method for preparing drug toxin PNU-159682 (morpholinyl anthracycline derivative) for antibody-conjugated drugs and intermediates involved in the preparation method. The preparation method of the present disclosure improves the stability, practicability and scalability of the process by introducing protecting groups and changing to reagents that are capable to amplify; the preparation method of the present disclosure minimizes the risk and operational difficulty during scale-up production; production and operation is simple and convenient.

## Description

### TECHNICAL FIELD

The present disclosure relates to the field of organic synthesis, in particular to a method (method for morpholinyl anthracycline derivatives) for preparing a drug toxin PNU-159682 for antibody drug conjugates and intermediates therein.

### BACKGROUND

Antibody-drug conjugate (ADC for short) is a new type of anti-tumor drug. Its principle is to connect cytotoxin to antibody. Through antibody recognition of specific antigen on the surface of cancer cell, it enters the cancer cells through endocytosis, thereby transporting cytotoxins to the target, achieve the purpose of targeted treatment of malignant tumors. Compared with traditional small-molecule anti-tumor drugs, ADC is more specific and effective because it can rely on the targeted recognition of antibodies and the high activity of toxins.

ADC includes three different components, namely antibody, linker and toxin. The antibody achieves targeting, the linker ensures the stability of the ADC during the blood transport process, and after reaching the target point, the toxin exerts a killing effect on cancer cells. According to the different mechanisms of action, toxins applicable for ADCs comprise microtubule inhibitors, DNA damaging agents, RNA polymerase inhibitors, etc. At present, the toxins used by ADCs commercially available and in clinical trials are mostly microtubule inhibitors, which mainly including dolastatin-based compounds, such as MMAE, MMAF and MMAD, and maytansine-based designed compounds, such as DM1 and DM4. In terms of linkers, the main applications are non-cleavable types, such as valine-citrulline (Valine-Citriline) and cyclohexyl carboxylic acid (MCC). After lysosomal hydrolysis, the drug is still active, and it binds to a certain amino acid residue through the linker

There are many ways to form antibody-drug conjugates. It can be formed either by chemically coupling the amino or sulfhydryl group on the antibody and the drug linker, or by modifying the antibody, then coupling with the drug linker for chemical reaction coupling or enzyme catalyzed reaction coupling after a specific functional group is introduced on the antibody. The molecular structure of the antibody drug toxin involved in the present disclosure is shown below.

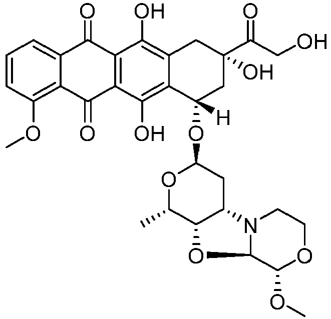

For the synthesis method of PNU-159682 currently reported in the literature, refer to WO2012/73217, 2012, A1. The synthesis method uses Nemorubicin to oxidize and cyclize without a protective group. The oxidant is DMDO (acetone peroxy), which is cumbersome and explosive, and the cyclization reagent is trichloroisocyanuric acid. The yield of cyclization is low and it is not easy to purify.

### SUMMARY

In the prior art, there are many defects such as the occurrence of the secondary reaction of primary alcohol being oxidized, which leads to the low target yield during the scale-up production. In view of the defects of the prior art, the present disclosure provides a novel synthesis method and intermediate compounds involved in each step of the synthesis method.

The above-mentioned objects of the present disclosure are achieved by the following technical solutions.

Dissolve a compound of structural formula 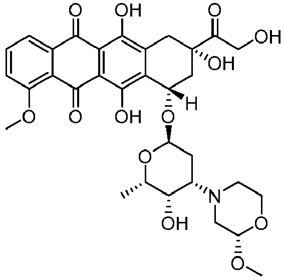 in solvent X, and substitute protective group R in the presence of reagent Y to obtain an intermediate compound of structural formula

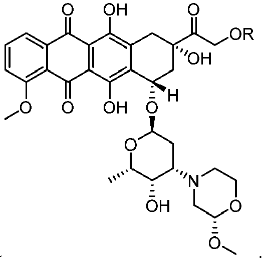

Wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv.

Solvent X is selected from a group consisting of dichloromethane, 1,2-dichloroethane, chloroform, carbon tetrachloride, acetonitrile, acetone, ethyl acetate, methyl acetate, water, benzene, ethyl ether, ethylene glycol dimethyl ether, methyl tert-butyl ether, diphenyl ether, 1,4-dioxane, N,N-dimethylformamide, N,N-dimethylacetamide, tetrahydrofuran, 2-methyltetrahydrofuran and a mixture thereof, preferably N, N-dimethylformamide;

The reaction reagent Y is selected from a group consisting of 4-dimethylaminopyridine, pyridine, imidazole, trimethylamine, triethylamine diisopropyldiamine and a mixture thereof, preferably imidazole;

Reaction temperature is 20 °C subzero to 80°C, preferably 20 to 25°C.

Dissolve the intermediate compound 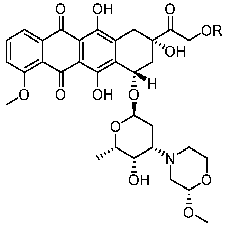 obtained in the previous step in an appropriate solvent M, add oxidizing reagent N, and obtain 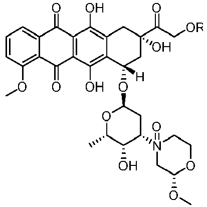 by reaction.

Preferably, the appropriate solvent M is selected from a group consisting of dichloromethane, 1,2-dichloroethane, chloroform, carbon tetrachloride, acetonitrile, acetone, ethyl acetate, methyl acetate, water, benzene, diethyl ether, ethylene glycol dimethyl ether, methyl tert-butyl ether, diphenyl ether, 1,4-dioxane, N,N-dimethylformamide, N,N-dimethylacetamide, tetrahydrofuran, 2-methyltetrahydrofuran, and a mixture thereof;

Preferably, the reagent N is selected from a group consisting of Jones reagent, Collins reagent (CrO3.2Py), pyridinium chlorochromate (PCC), pyridine dichromate (PDC), manganese dioxide, DMSO, Dess-Martin Oxidizer, potassium permanganate, periodic acid, osmium tetroxide, 30% hydrogen peroxide, m-chloroperoxybenzoic acid m-CPBA, tert-butyl hydroperoxide TBHP, acetone peroxy (DMDO) and a mixture thereof.

Preferably, the reagent N is tert-butyl hydroperoxide TBHP, acetone peroxy (DMDO) or m-chloroperoxybenzoic acid m-CPBA, and the solvent M is dichloromethane;

In this step, the reaction temperature is 50°C subzero to 50°C, preferably 40°C subzero to 25°C.

Preferably, this step further comprises a step of separating 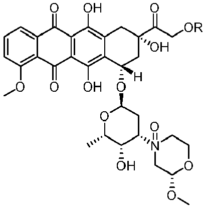 from the reaction liquid after the reaction is complete.

Preferably, the separation comprises evaporating solvent under reduced pressure, and then purifying or recrystallizing by medium pressure chromatography to obtain

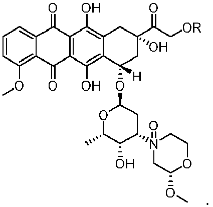

The present disclosure also provides an intermediate compound of structural formula 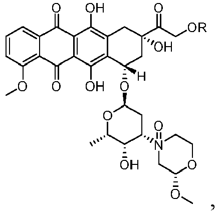 wherein R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-Ethoxyethyl EE, 2-(Trimethylsilyl)ethoxymethyl SEM, Allyl, acetyl Ac, benzoyl Bz, pivaloyl Pv and a mixture thereof; preferably the following compounds: 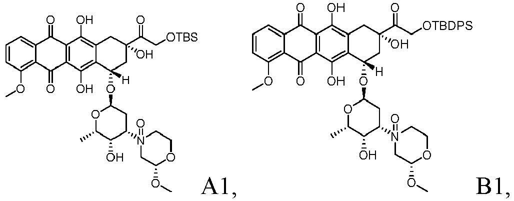 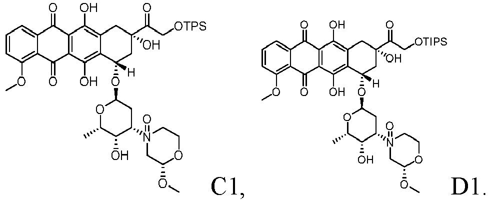 The compound 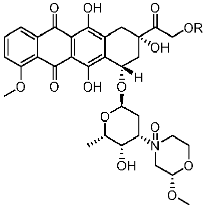 is a new compound that has never been reported, and we used a inventive design in the synthesis.

Dissolve the intermediate compound 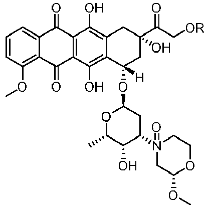 obtained in the previous step in an appropriate solvent O, add the dehydrating reagent P, and obtain 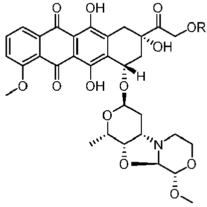 by reaction.

Preferably, the solvent O is selected from a group consisting of dichloromethane, 1,2-dichloroethane, chloroform, carbon tetrachloride, acetonitrile, acetone, ethyl acetate, methyl acetate, benzene, toluene, mesitylene, xylene, chlorobenzene, diethyl ether, ethylene glycol dimethyl ether, methyl tert-butyl ether, diphenyl ether, 1,4-dioxane, N, N-dimethylformamide, N, N-Dimethylacetamide, tetrahydrofuran, 2-methyltetrahydrofuran and a mixture thereof.

Preferably, the reagent P is selected from a group consisting of dicyclohexylcarbodiimide (DCC), polyphosphoric acid, Burgess reagent, bis[a,a-bis(trifluoromethyl)phenethyl alcohol]-diphenyl sulfide, carbon disulfide, methyl iodide, sodium hydroxide, potassium hydroxide, lithium hydroxide, sodium hydride, potassium tert-butoxide, sodium tert-butoxide, sodium methoxide, sodium ethoxide, n-butyl lithium, lithium diisopropylamide, cyanuric chlorid and a mixture thereof.

Preferably, the reagent P is dicyclohexylcarbodiimide (DCC), polyphosphoric acid, Burgess reagent or bis[a,a-bis(trifluoromethyl)phenethyl alcohol]-diphenylsulfide, the solvent O is dichloromethane.

In this step, the reaction temperature is 78°C subzero to 25°C, preferably the reaction temperature is 40°C subzero to 25°C.

Preferably, this step further comprises a step of separating 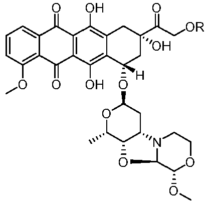 from the reaction liquid after the reaction is complete.

Preferably, the separation comprises evaporating solvent under reduced pressure, and then purifying or recrystallizing by medium-pressure chromatography to obtain

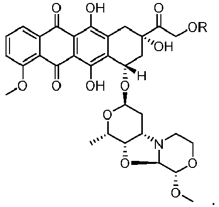

The present disclosure also provides an intermediate compound of structural formula 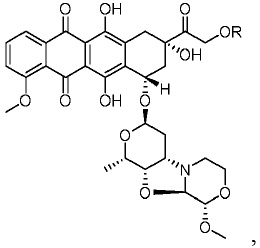 which is obtained by dehydration and cyclization reaction of compound 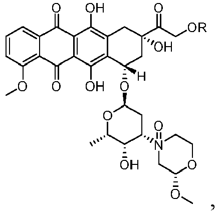 wherein R is selected from a group consisting of trimethylsilyl TMS, tert-butyldimethylsilyl TBS, and tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr , 2-tetrahydropyranyl THP, methoxymethyl MOM, 2-ethoxyethyl EE, 2-(trimethylsilyl)ethoxymethyl SEM, Allyl, acetyl Ac, benzoyl Bz, pivaloyl Pv a mixture thereof; the compound 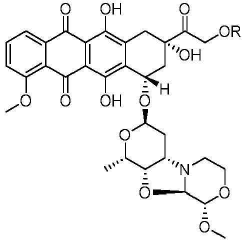 is a new compound that has never been reported, and we have used inventive designs in the synthesis.

Dissolve the compound 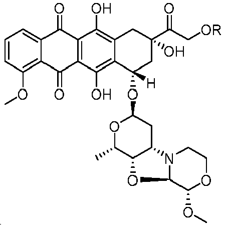 in an appropriate solvent Q, add the deprotection reagent S, and obtain 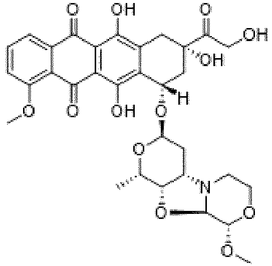 by the deprotection reaction.

Wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, pivaloyl Pv and a mixture thereof.

The reagent S is selected from a group consisting of hydrochloric acid methanol solution, hydrochloric acid ethanol solution, hydrochloric acid 1,4-dioxane solution, hydrochloric acid ether solution, hydrochloric acid tetrahydrofuran, tetrahydrofuran acetate solution, tetramethylammonium fluoride, tetraethylammonium fluoride, tetra-n-butylammonium fluoride (TBAF), Pd/C catalytic hydrogenation, DDQ, p-toluenesulfonic acid, methanol/sodium hydroxide, methanol/sodium methoxide.

The solvent Q is selected from a group consisting of dichloromethane, 1,2-dichloroethane, chloroform, carbon tetrachloride, acetonitrile, methanol, ethanol, acetone, ethyl acetate, methyl acetate, benzene, toluene, mesitylene, xylene, chlorobenzene, ethyl ether, ethylene glycol dimethyl ether, methyl tert-butyl ether, diphenyl ether, 1,4-dioxane, N, N-dimethylformamide, N, N dimethylacetamide, tetrahydrofuran, 2-methyltetrahydrofuran and a mixture thereof.

Preferably, in this step, the reaction temperature is 50°C subzero to 50°C, preferably 30°C subzero to 25°C.

Preferably, in this step, reagent S is tetrabutylammonium fluoride (TBAF), and reagent Q is tetrahydrofuran.

Preferably, in this step, separate compound 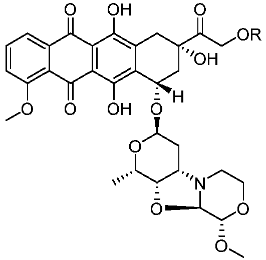 from the reaction liquid after the reaction is complete.

Preferably, the separation comprises evaporating solvent under reduced pressure, and then purifying or recrystallizing by medium-pressure chromatography to obtain

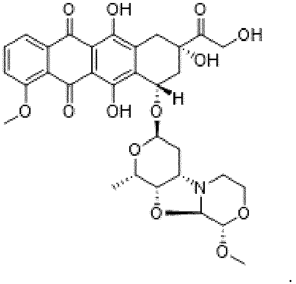

The preparation method of the present disclosure does not directly use the compound Nemorubicin with structural formula 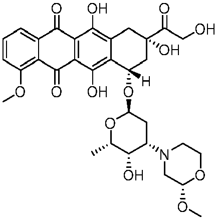 to synthesize PNU-159682, which improves the effectiveness of the N atom reaction, thereby effectively controlling the occurrence of side reactions of primary alcohols being oxidized; minimizing the risk and difficulty in scale-up production; no reverse phase preparation is required, and the preparation and production operation is simple. As mentioned above, this method minimizes the difficulty of operation, makes the quality standard easier to control, and can be applied to the preparation kilogram level. The preparation method of the present disclosure improves the stability, practicability and scalability of the process by introducing protective groups and changing to reagents that are easy to scale up.

As used in this context, the definitions of commonly used organic abbreviations and their corresponding CAS numbers are shown in Table 1:

**Table1**

| Abbreviat ion | Definition | CAS No. |
|---|---|---|
| BrOP | Bromotris(dimethylamino)phosphorus hexafluorophosphate | 50296-37-2 |
| DBU | 1,8-diazabicyclo[5.4.0]undec-7-ene | 6674-22-2 |
| DECP | Diethyl cyanophosphate | 2942-58-7 |
| DIEA | N,N-Diisopropylethylamine | 7087-68-5 |
| DMT | Dimethyl Val-Val-Dil-OH | 133120-89-5 |
| HOSu | N-hydroxysuccinimide | 6066-82-6 |
| TEA | Triethylamine | 121-44-8 |
| DCC | N,N'-Dicyclohexylcarbodiimide | 538-75-0 |
| EDCI | 1-(3-Dimethylaminopropyl)-3-ethylcarbodiimide hydrochloride | 7084-11-9 |
| DIC | N,N'-Diisopropylcarbodiimide | 693-13-0 |
| HATU | 2-(7-Azobenzotriazole)-N,N,N',N'-tetramethylurea hexafluorophosphate | 148893-10-1 |
| HBTU | Benzotriazole-N,N,N',N'-tetramethylurea hexafluorophosphate | 94790-37-1 |
| HBPIPU | (Benzotriazol-1-yloxy)dipiperidine carbohexafluorophosphate | 206752-41-2 |
| HBPyU | O-(benzotriazol-1-yl)-N,N,N',N'-dipyrrolylurea hexafluorophosphate | 105379-24-6 |
| HSPyU | Dipyrrolidinyl (N-succinimidyloxy) hexafluorophosphate | 207683-26-9 |
| HCTU | 6-Chlorobenzotriazole-1,1,3,3-tetramethylurea hexafluorophosphate | 330645-87-9 |
| HOTU | O-[(Ethoxycarbonyl)cyanomethylamine]-N,N,N',N'-tetramethylt | 333717-40-1 |
| | hiourea hexafluorophosphate | |
| HOTT | N,N,N',N'-Tetramethyl-S-(1-oxo-2-pyridyl)thiourea hexafluorophosphate | 212333-72-7 |
| HSTU | N,N,N',N'-tetramethylurea-O-(N-succinimidyl) hexafluorophosphate | 265651-18-1 |
| HDMA | 1-[(Dimethylamino)(morpholine)methyl]-3-oxo-1H-[1,2,3]triazol e[4,5-b]pyridine3-hexafluorophosphate | 958029-37-3 |
| TATU | 2-(7-Azabenzotriazole)-N,N,N',N'-tetramethylurea tetrafluoroborate | 873798-09-5 |
| TBTU | O-benzotriazole-N,N,N',N'-tetramethylurea tetrafluoroborate | 125700-67-6 |
| TCTU | O-(6-Chloro-1H-benzotriazol-1-yl)-N,N,N',N'-tetramethylurea tetrafluoroborate | 330641-16-2 |
| TCFH | N,N,N',N'-Tetramethylchloroformamidine hexafluorophosphate | 94790-35-9 |
| TDBTU | N,N,N',N'-tetramethyl-O-(4-carbonyl-3,4-dihydro-1,2,3-benzotri azin-3-yl)urea tetrafluoroborate | 125700-69-8 |
| TOTU | O-[(Ethoxycarbonyl)cyanomethylamine]-N,N,N',N'-tetramethylt hiourea tetrafluoroboron | 136849-72-4 |
| TOTT | 2-(1-Pyridin-2-yl oxide)-1,1,3,3-Tetramethylisothiourea tetrafluoroborate | 255825-38-8 |
| TPTU | 2-(2-pyridone-1-yl)-1,1,3,3-tetramethylurea tetrafluoroborate | 125700-71-2 |
| TFFH | Fluoro-N,N,N',N'-tetramethylurea hexafluorophosphate | 164298-23-1 |
| BTFFH | N,N,N',N'-bis(tetramethylene)fluoroformamidine hexafluorophosphate | 164298-25-3 |
| TNTU | 2-(Endo-5-norbornene-2,3-dicarboximide)-1,1,3,3-tetramethylure a tetrafluoroborate | 125700-73-4 |
| TSTU | 2-succinimidyl-1,1,3,3-tetramethylurea tetrafluoroborate | 105832-38-0 |
| COMU | cycluron | 2163-69-1 |
| T3P | Propyl phosphate tricyclic anhydride | 68957-94-8 |
| BOP | 1H-benzotriazol-1-yloxotris(dimethylamino)phosphonium | 56602-33-6 |
| | hexafluorophosphate | |
| PyBOP | 1H-benzotriazol-1-yloxytripyrrolidinyl hexafluorophosphate | 128625-52-5 |
| PyBrOP | Tripyrrolidinylphosphonium bromide hexafluorophosphate | 132705-51-2 |
| PyClOP | Chlorotripyrrolidinyl hexafluorophosphate | 133894-48-1 |
| BrOP | Bromotris(dimethylamino)phosphonium hexafluorophosphate | 50296-37-2 |
| PyAOP | (3H-1,2,3-Triazolo[4,5-b]pyridin-3-oxy)tris-1-pyrrolidinyl hexafluorophosphate | 156311-83-0 |
| PyCIU | 1-(Chloro-1-pyrrolidinylmethylene)pyrrolidine hexafluorophosphate | 135540-11- 3 |
| CDI | N,N'-Carbonyl Diimidazole | 530-62-1 |
| TsIm | 1-p-toluenesulfonyl imidazole | 2232-08-8 |
| TPSI | 1-(2,4,6-triisopropylphenylsulfonyl)imidazole | 50257-40-4 |
| TSTU | 2-succinimidyl-1,1,3,3-tetramethylurea tetrafluoroborate | 105832-38-0 |
| DEPBT | 3-(diethoxy o-acyloxy)-1,2,3-benzotriazin-4-one | 165534-43-0 |
| DMTMM | 4-(4,6-Dimethoxytriazin-2-yl)-4-methylmorpholine hydrochloride | 3945-69-5 |
| EEDQ | 2-ethoxy-1-ethoxycarbonyl-1,2-dihydroquinoline | 16357-59-8 |
| CIP | 2-chloro-1,3-dimethylimidazolium hexafluorophosphate | 101385-69-7 |
| CIB | 2-chloro-1,3-dimethylimidazolium tetrafluoroborate | 153433-26-2 |
| DMC | 2-chloro-1,3-dimethylimidazolium chloride | 37091-73-9 |
| EDC | 1-(3-Dimethylaminopropyl)-3 -ethylcarbodiimide hydrochloride | 7084-11-9 |
| DIC | N,N'-Diisopropylcarbodiimide | 693-13-0 |
| HOAt | N-hydroxy-7-azabenzotriazole | 39968-33-7 |
| HOBt | 1-hydroxybenzotriazole | 2592-95-2 |

### BRIEF DESCRIPTION OF THE DRAWINGS

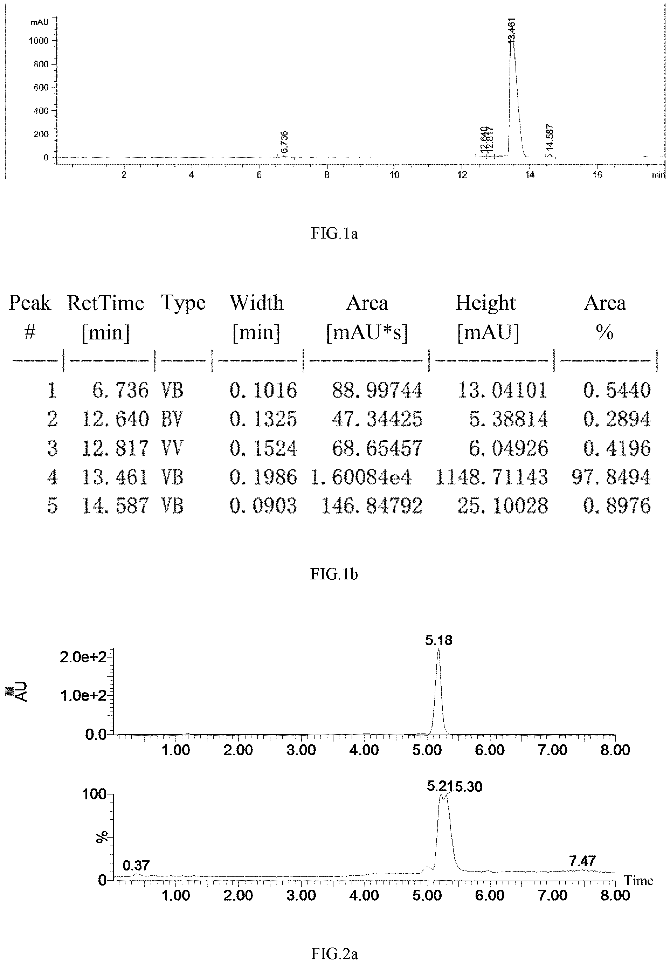

Figure 1a is a liquid chromatogram of compound A1 synthesized in the present disclosure.
Figure 1b is a graph of liquid chromatography data of compound A1 synthesized in the present disclosure.
Figure 2a is a gas chromatogram of compound A1 synthesized in the present disclosure.

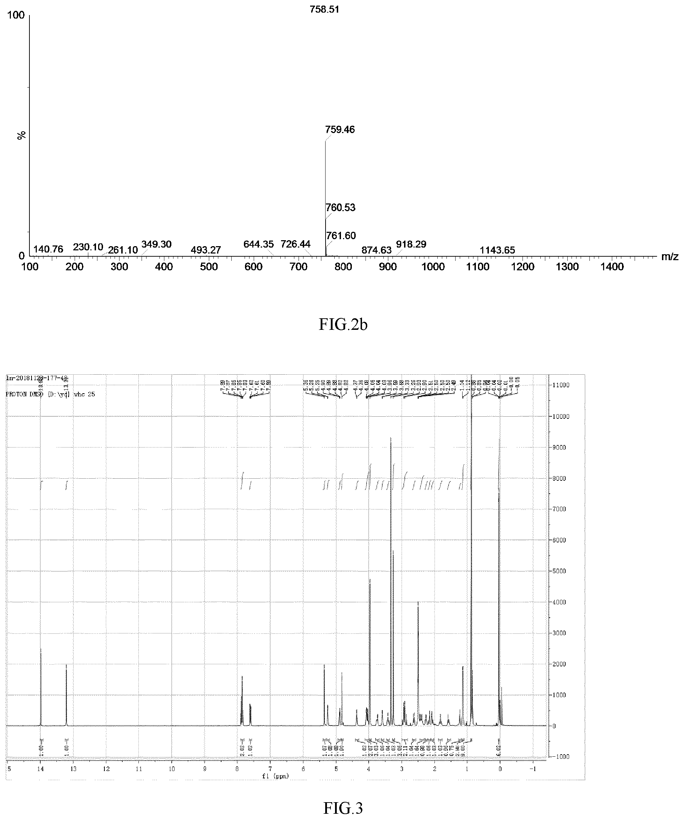

Figure 2b is a mass spectrum of compound A1 synthesized in the present disclosure.
Figure 3 is a NMR spectrum of compound A1 synthesized by the present disclosure.

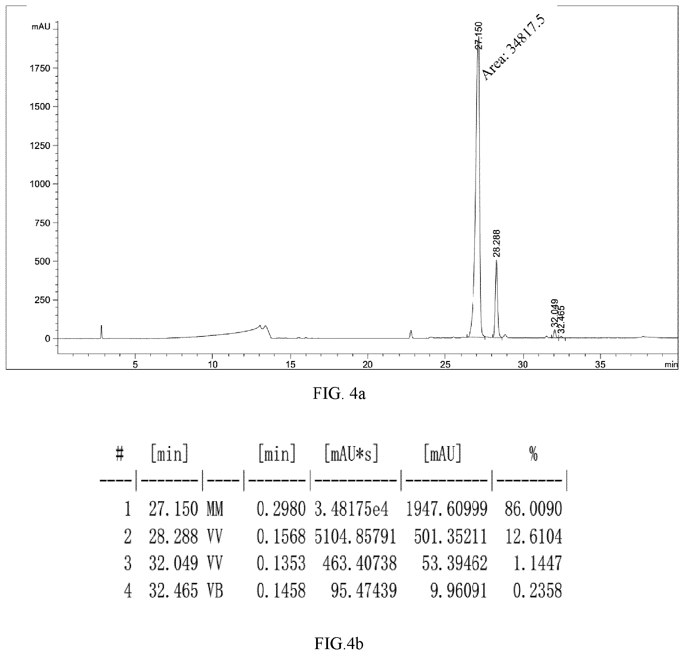

Figure 4a is a liquid chromatogram of compound A2 synthesized in the present disclosure.
Figure 4b is a graph of liquid chromatographic data of compound A2 synthesized in the present disclosure.

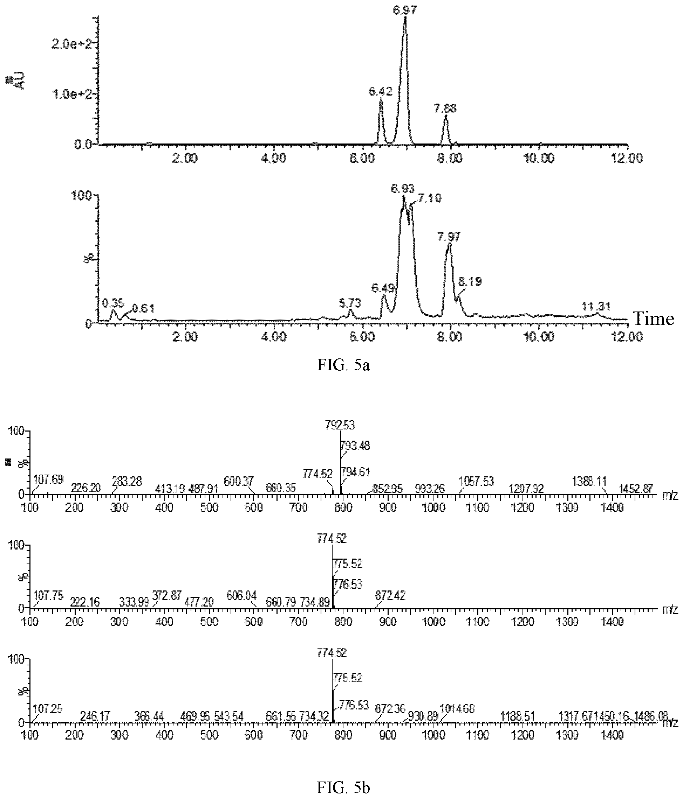

Figure 5a is a gas chromatogram of compound A2 synthesized in the present disclosure.
Figure 5b is a mass spectrum of compound A2 synthesized in the present disclosure.

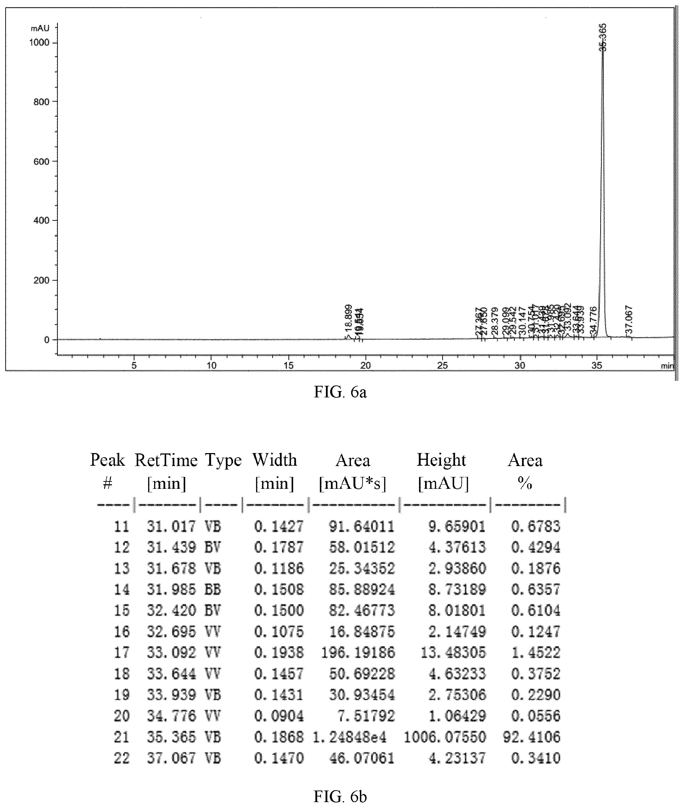

Figure 6a is a liquid chromatogram of compound A3 synthesized in the present disclosure.
Figure 6b is a graph of liquid chromatography data of compound A3 synthesized in the present disclosure.

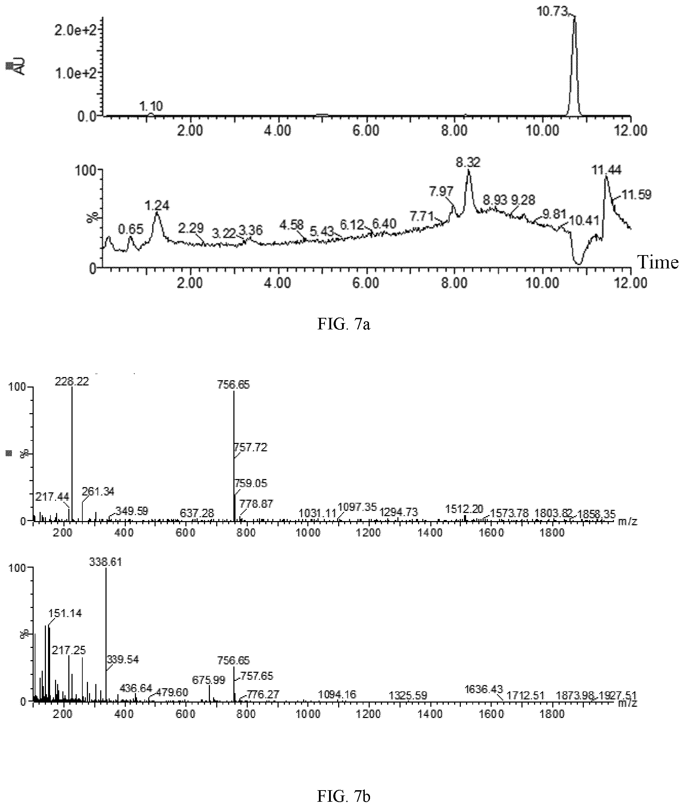

Figure 7a is a gas chromatogram of compound A3 synthesized in the present disclosure.
Figure 7b is a mass spectrum of compound A3 synthesized in the present disclosure.

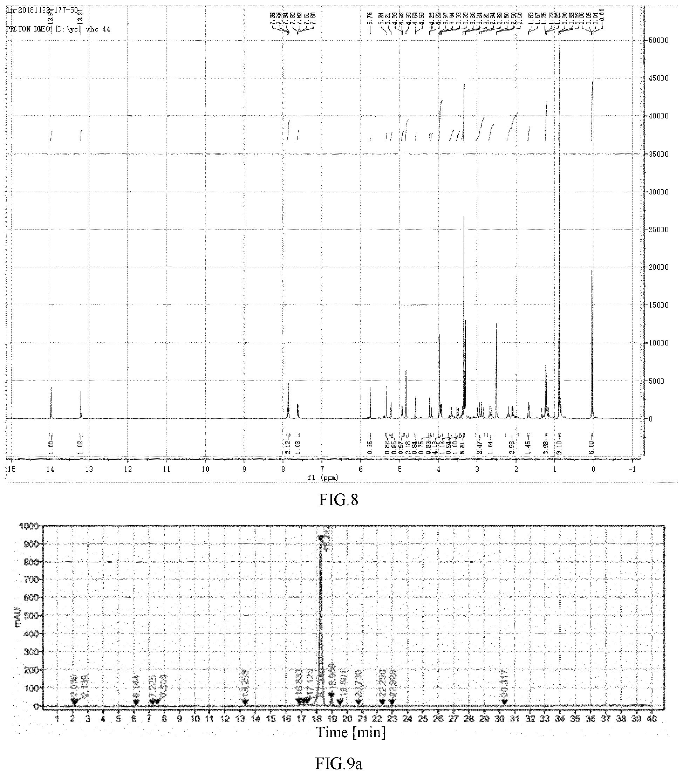

Figure 8 is a NMR spectrum of compound A3 synthesized in the present disclosure.
Figure 9a is a liquid chromatogram of the compound PNU-159682 synthesized in the present disclosure.

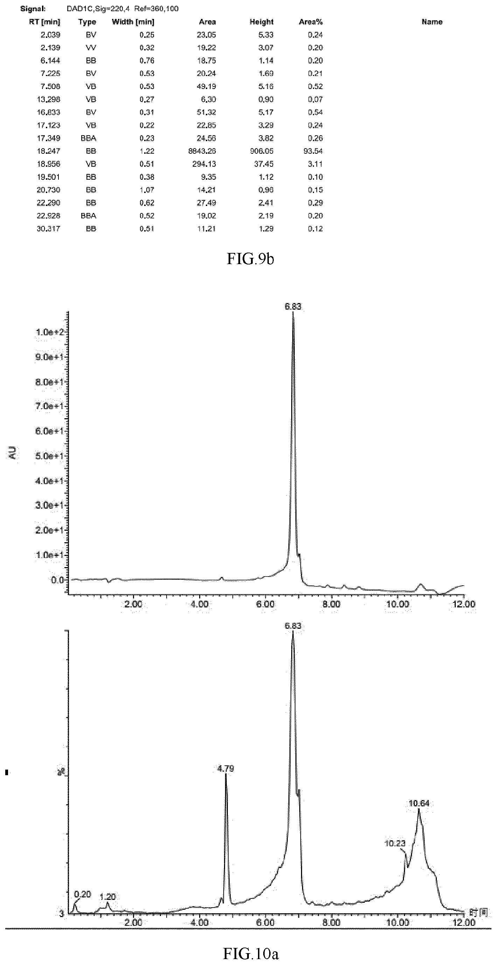

Figure 9b is a graph of liquid chromatographic data of the compound PNU-159682 synthesized in the present disclosure.
Figure 10a is a gas chromatogram of the compound PNU-159682 synthesized in the present disclosure.

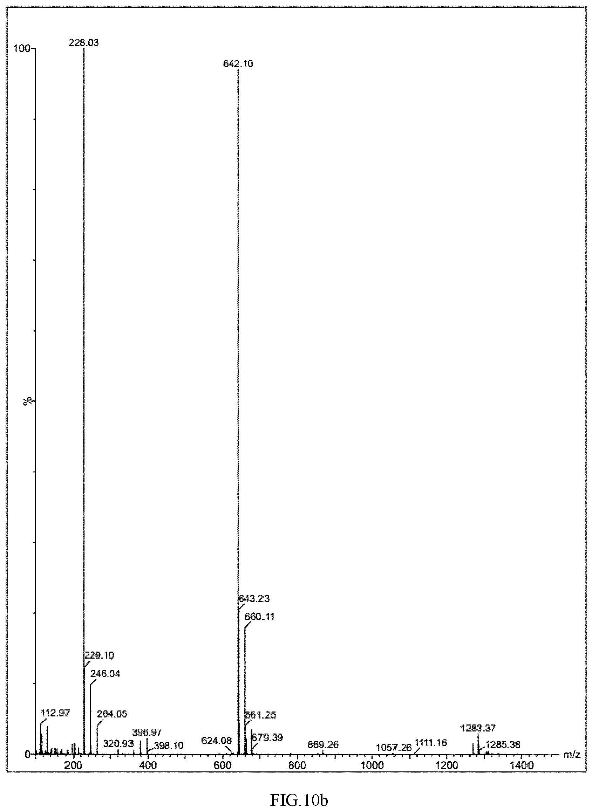

Figure 10b is a mass spectrum of the compound PNU-159682 synthesized in the present disclosure.

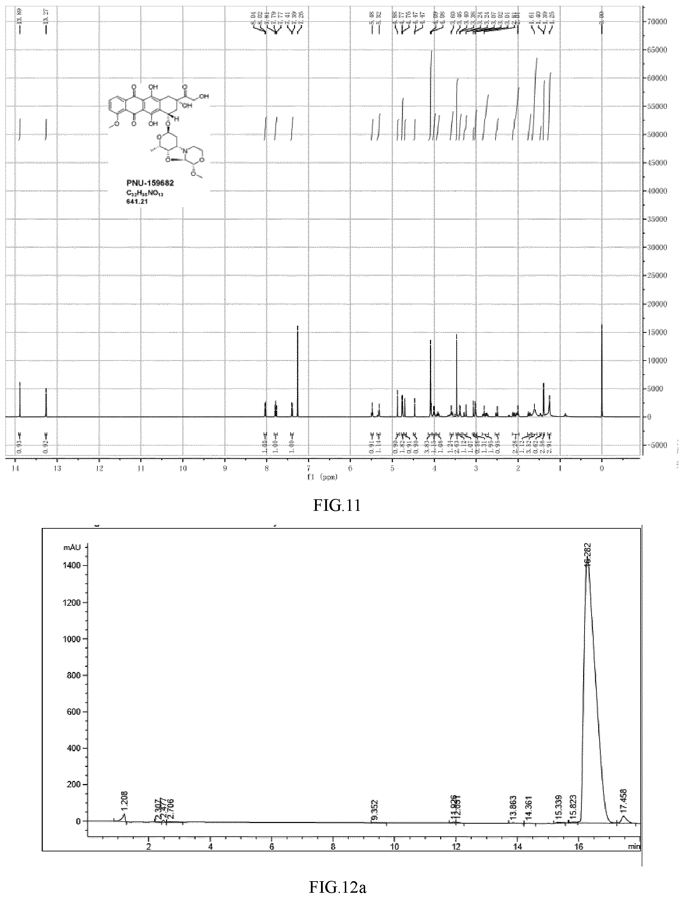

Figure 11 is a NMR spectrum of the compound PNU-159682 synthesized in the present disclosure.
Figure 12a is a liquid chromatogram of compound B1 synthesized in the present disclosure.

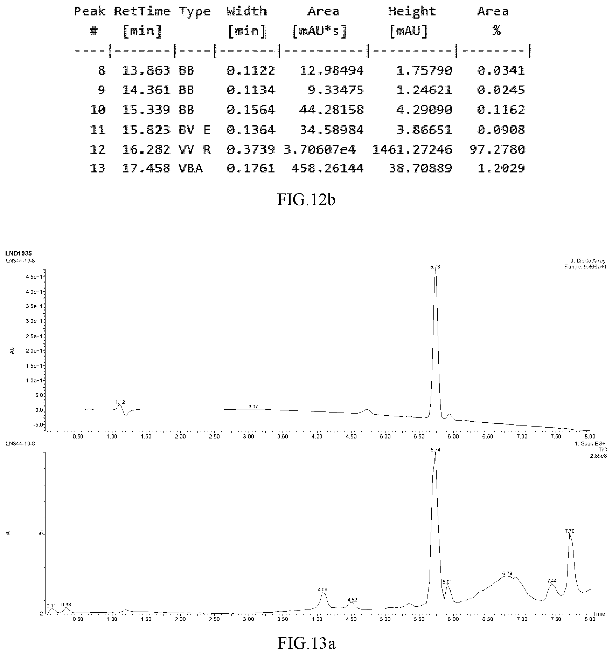

Figure 12b is a graph of liquid chromatographic data of compound B1 synthesized in the present disclosure.
Figure 13a is a gas chromatogram of compound B1 synthesized in the present disclosure.

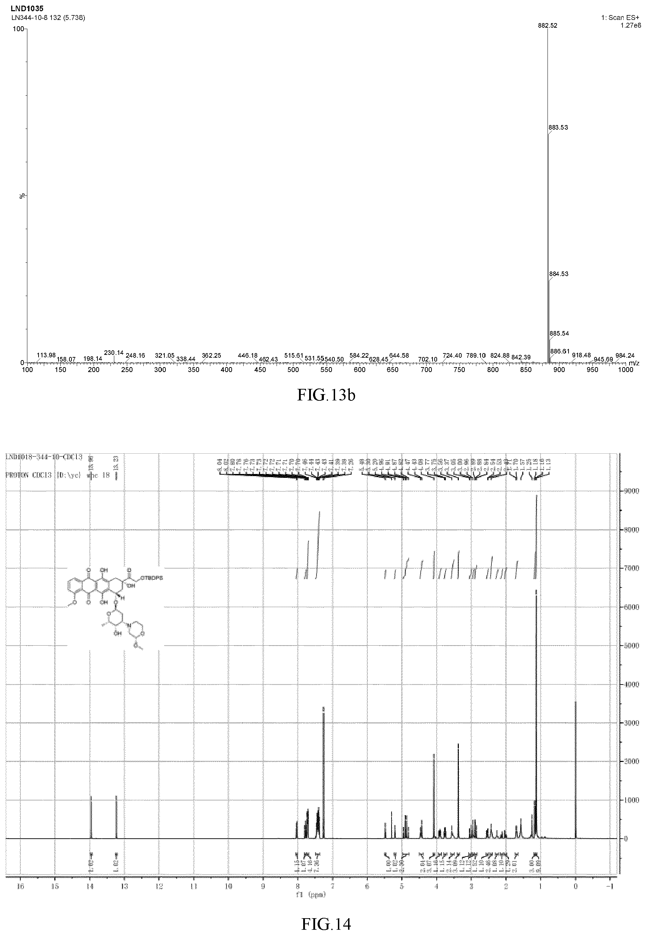

Figure 13b is a mass spectrum of compound B 1 synthesized in the present disclosure.
Figure 14 is a NMR spectrum of compound B 1 synthesized in the present disclosure.

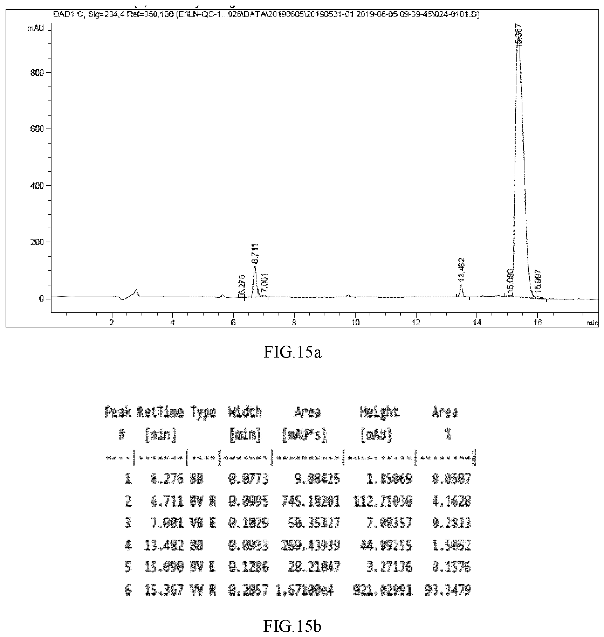

Figure 15a is a liquid chromatogram of compound C1 synthesized in the present disclosure.
Figure 15b is a graph of liquid chromatography data of compound C1 synthesized in the present disclosure.

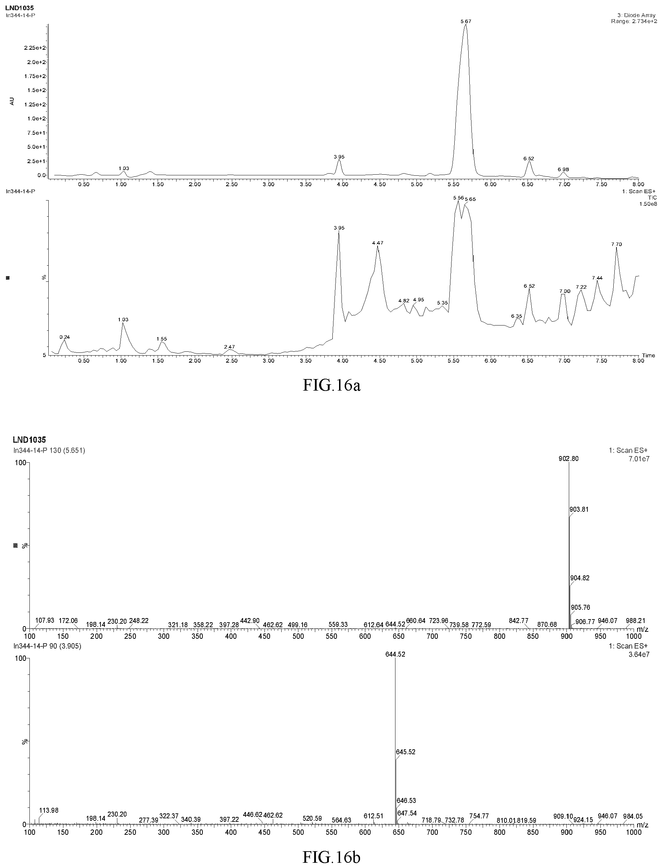

Figure 16a is a gas chromatogram of compound C1 synthesized in the present disclosure.
Figure 16b is a mass spectrum of compound C1 synthesized in the present disclosure.

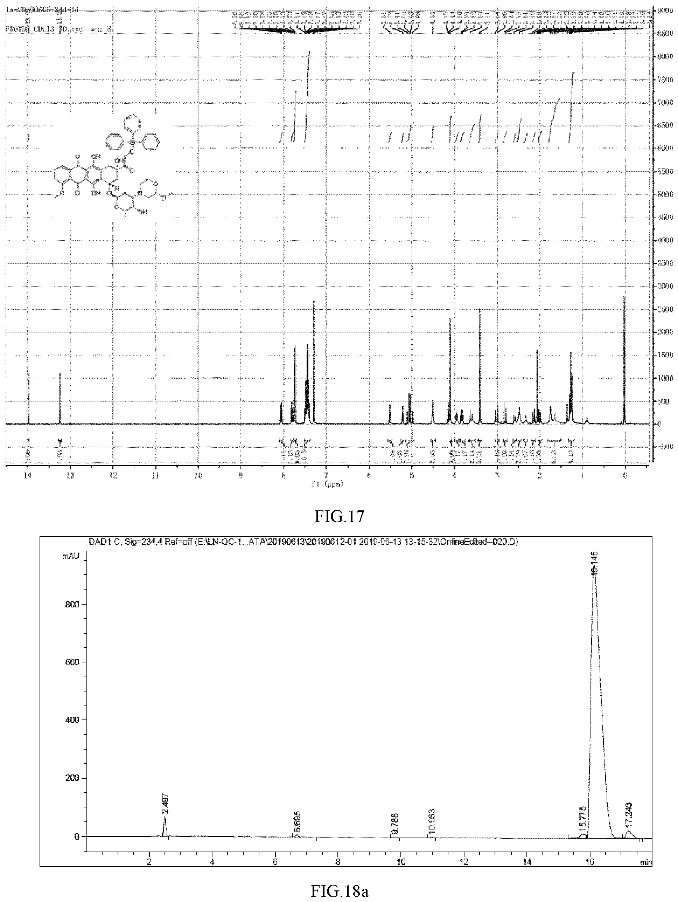

Figure 17 is a NMR spectrum of compound C1 synthesized in the present disclosure.
Figure 18a is a liquid chromatogram of compound D1 synthesized in the present disclosure.

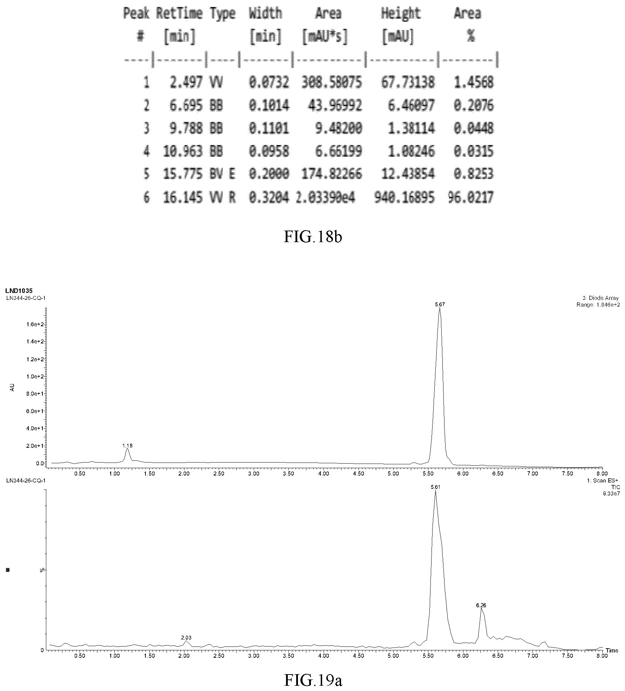

Figure 18b is a graph of liquid chromatography data of compound D1 synthesized in the present disclosure.
Figure 19a is a gas chromatogram of compound D1 synthesized in the present disclosure.

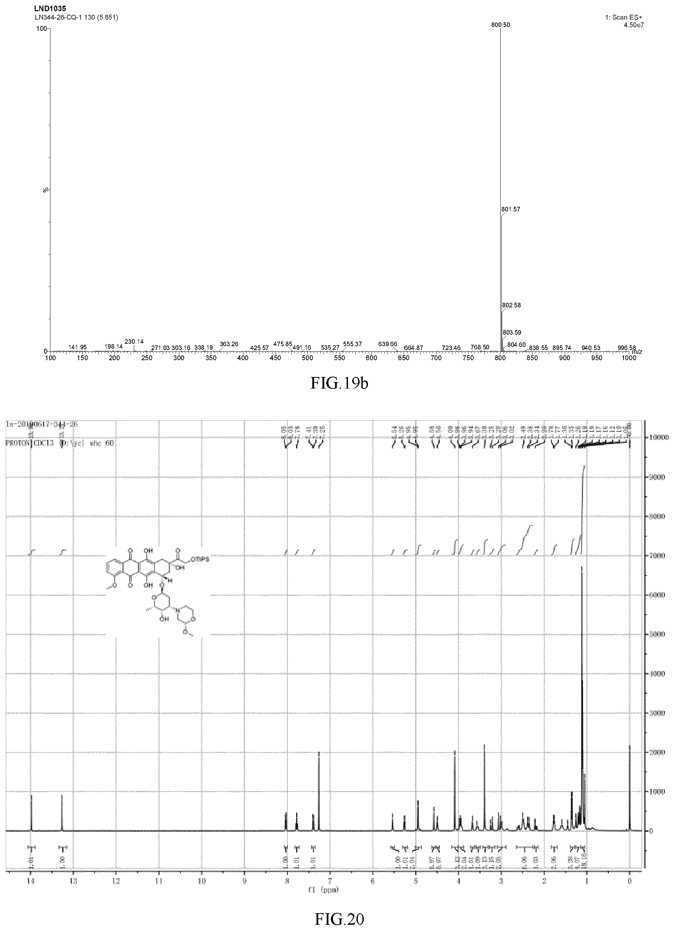

Figure 19b is a mass spectrum of compound D1 synthesized in the present disclosure.
Figure 20 is a NMR spectrum of compound D1 synthesized in the present disclosure.

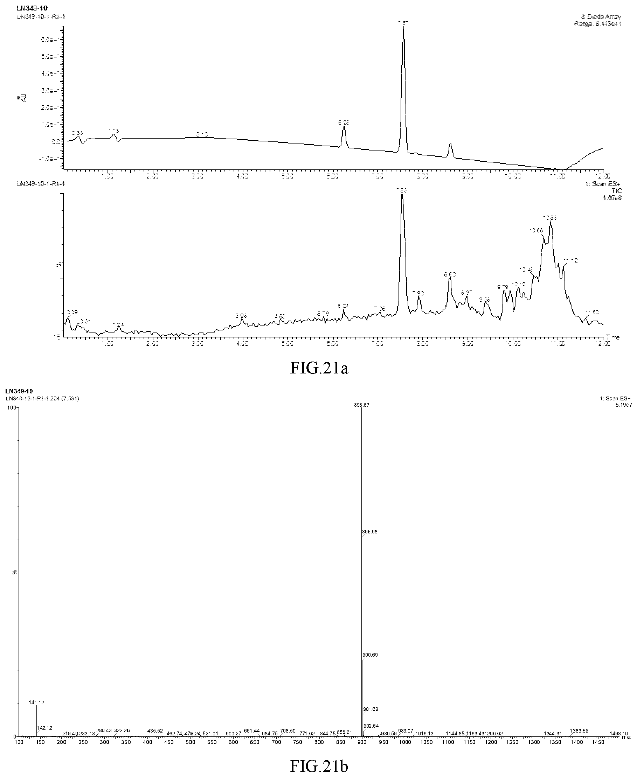

Figure 21a is a gas chromatogram of compound B2 synthesized in the present disclosure.
Figure 21b is a mass spectrum of compound B2 synthesized in the present disclosure.

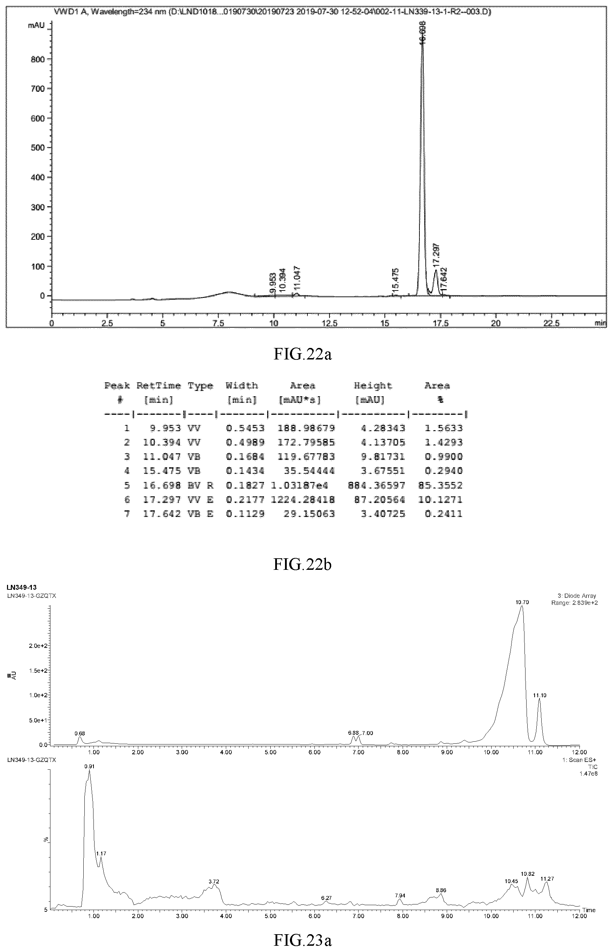

Figure 22a is a liquid chromatogram of compound B2 synthesized in the present disclosure.
Figure 22b is a graph of liquid chromatography data of compound B2 synthesized in the present disclosure.
Figure 23a is a gas chromatogram of compound B3 synthesized in the present disclosure.

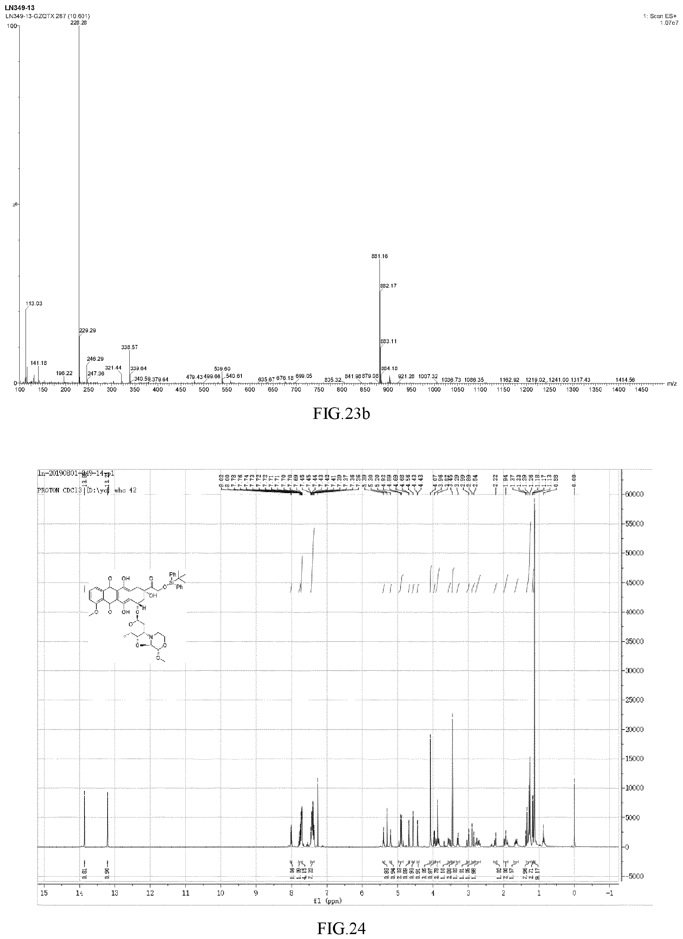

Figure 23b is a mass spectrum of compound B3 synthesized in the present disclosure.
Figure 24 is a NMR spectrum of compound B3 synthesized in the present disclosure.

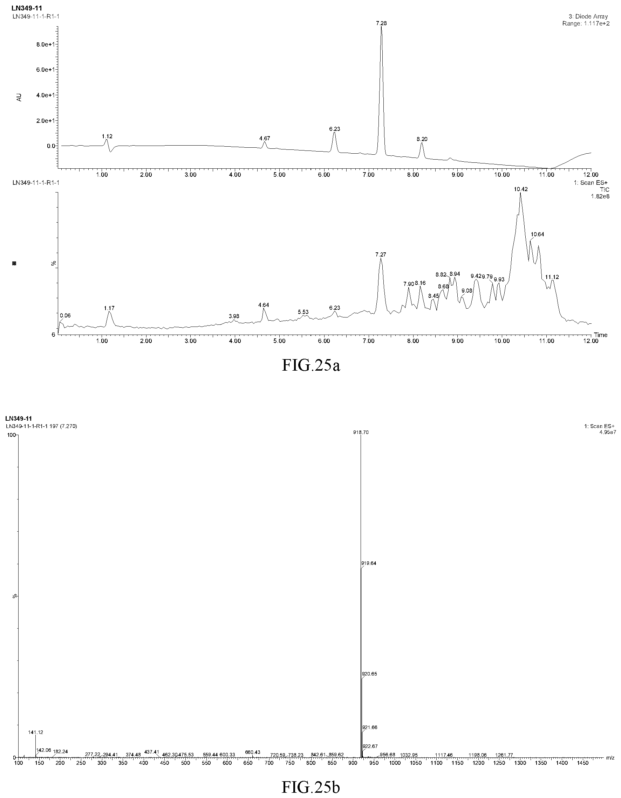

Figure 25a is a gas chromatogram of compound C2 synthesized in the present disclosure.
Figure 25b is a mass spectrum of compound C2 synthesized in the present disclosure.

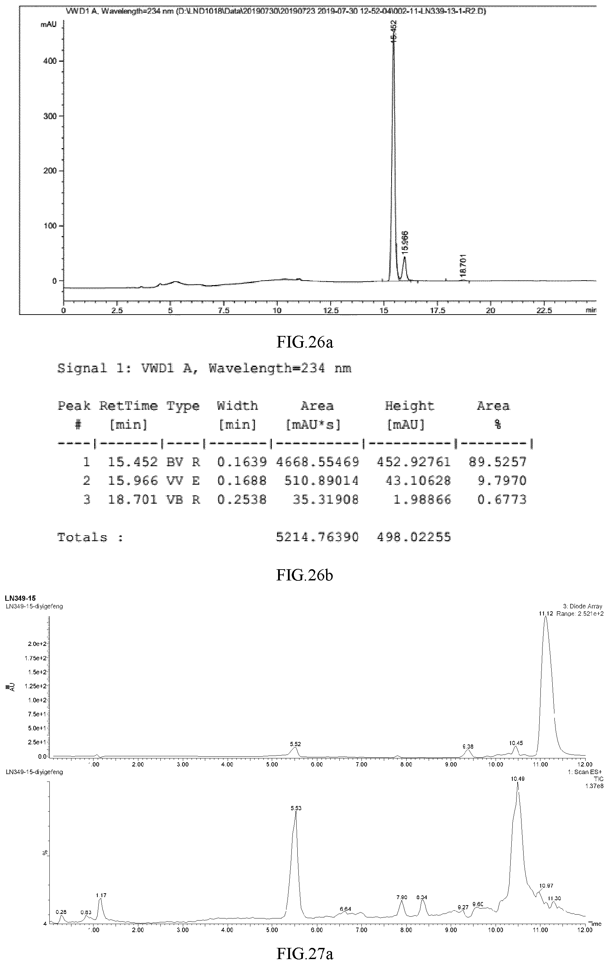

Figure 26a is a liquid chromatogram of compound C2 synthesized in the present disclosure.
Figure 26b is a graph of liquid chromatography data of compound C2 synthesized in the present disclosure.
Figure 27a is a gas chromatogram of compound C3 synthesized in the present disclosure.

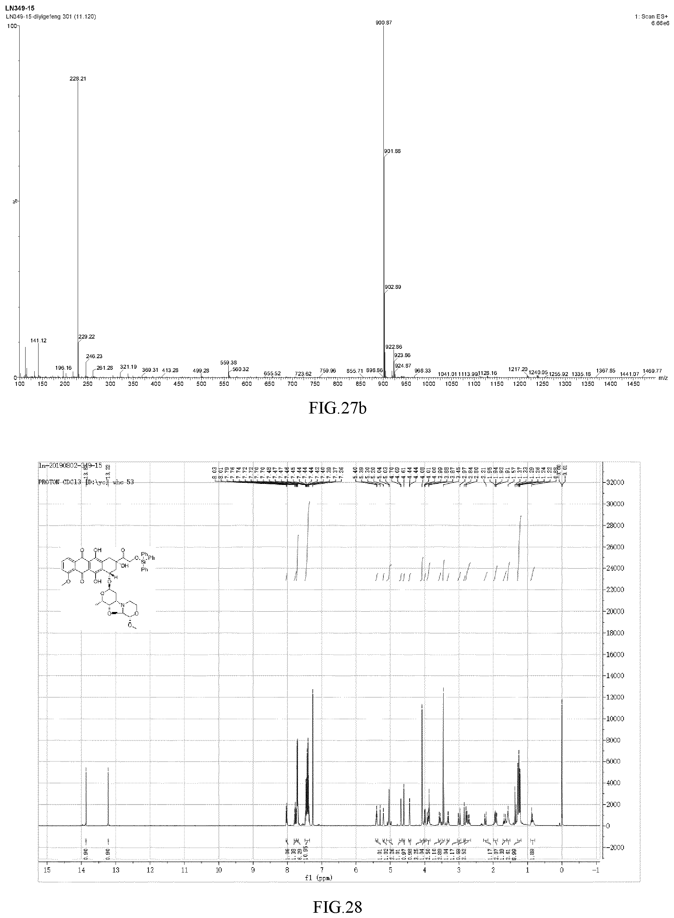

Figure 27b is a mass spectrum of compound C3 synthesized in the present disclosure.
Figure 28 is a NMR spectrum of compound C3 synthesized in the present disclosure.

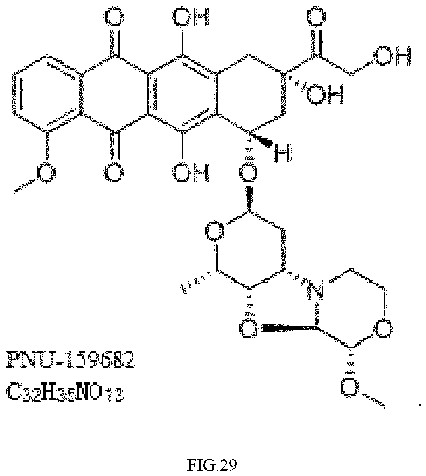

Figure 29 is a molecular structure diagram of compound PNU-159682 synthesized in the present disclosure.

### DESCRIPTION OF THE EMBODIMENTS

The technical solution of the present disclosure will be further non-restrictively described in detail below with reference to specific embodiments. It should be pointed out that the following embodiments are merely to illustrate the technical concept and features of the present disclosure, and their purpose is to enable those people familiar with the technology to understand the content of the present disclosure and implement them accordingly, but should not limit the protection scope of the present disclosure. All equivalent variations or modifications made according to the spirit of the present disclosure should be covered by the protection scope of the present disclosure.

LCMS refers to liquid chromatography mass spectrometry method; HPLC refers to high-performance liquid chromatography detection.

The raw materials and reagents for the reaction involved in the present disclosure are commercially available or prepared according to the method of the present disclosure.

The present disclosure provides a method for synthesizing PNU-159682, which includes the following steps:

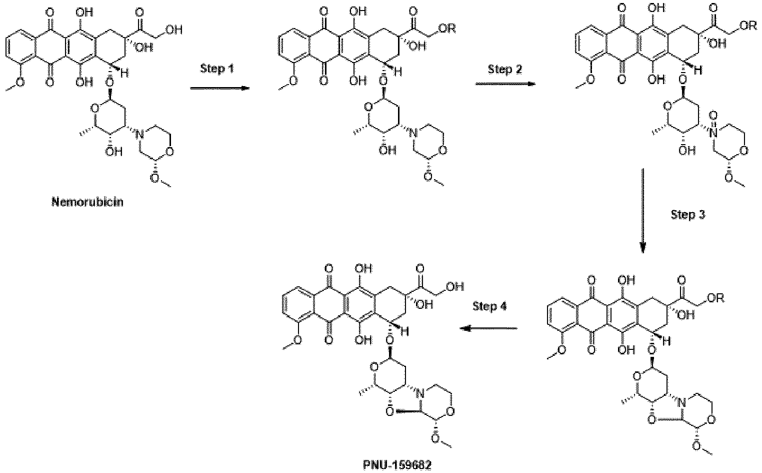

First, dissolve the compound (nemorubicin) of structural formula 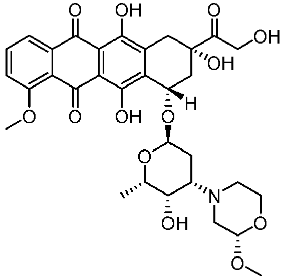 in solvent X, which is selected from a group consisting of dichloromethane, 1,2-dichloroethane, chloroform, carbon tetrachloride, acetonitrile, acetone, ethyl acetate, methyl acetate, water, benzene, ethyl ether, ethylene glycol dimethyl ether, methyl tert-butyl ether, diphenyl ether, 1,4-dioxane, N ,N-Dimethylformamide, N,N-dimethylacetamide, tetrahydrofuran, 2-methyltetrahydrofuran and a mixture thereof, in the presence of one or more of 4-dimethylaminopyridine, pyridine, imidazole, trimethylamine, triethylamine and diisopropyl diamine, it reacts with the substitution reagent with a protective group R, that is, it is substituted with the protective group R to obtain an intermediate compound of the structural formula 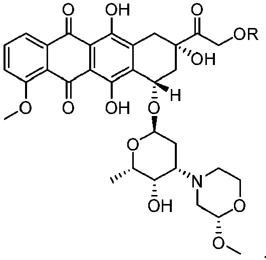 The protective group R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethyl Silyl Mes, benzyl Bn, p-methoxybenzyl PMB, trityl Tr, 2-tetrahydropyranyl THP, methoxymethyl MOM, 2-ethoxyethyl EE, 2-(Trimethylsilyl)ethoxymethyl SEM, Allyl, acetyl Ac, benzoyl Bz, pivaloyl Pv; the reaction temperature of the substitution reaction in this step is 20°C subzero to 80°C, Preferably it is 20 to 25°C.

Dissolve the compound 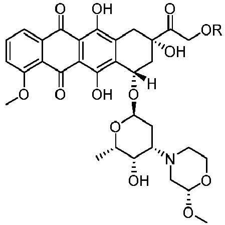 obtained in the previous step in an appropriate solvent M, which is selected from a group consisting of dichloromethane, 1,2-dichloroethane, chloroform, carbon tetrachloride, acetonitrile, acetone, ethyl acetate, methyl acetate, water, benzene, ethyl ether, ethylene glycol dimethyl ether, methyl tert-butyl ether, diphenyl ether, 1,4-dioxane, N,N-dimethylformamide, N,N- dimethylacetamide, tetrahydrofuran, 2-methyltetrahydrofuran and a mixture thereof, adding oxidizing reagent N, reagent N is selected from a group consisting of Jones reagent, Collins reagent (CrO3.2Py), pyridinium chlorochromate (PCC), pyridine dichromate (PDC), manganese dioxide, DMSO, Dess-Martin oxidant, potassium permanganate, periodic acid, osmium tetroxide, 30% hydrogen peroxide, m-chloroperoxybenzoic acid m-CPBA, tert-butyl hydroperoxide TBHP, acetone peroxy (DMDO) and mixture thereof, 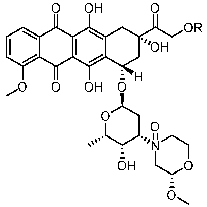 is obtained by oxidation reaction, the oxidation reaction temperature is 50°C subzero to 50°C, preferably 40°C subzero to 25°C. After the reaction is complete, separate the reaction solution, evaporate the solvent under reduced pressure, and then purify or recrystallize by medium pressure chromatography to obtain

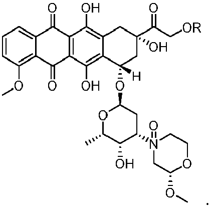

The obtained compound is dissolved in an appropriate solvent O, which is selected from a group consisting of dichloromethane, 1,2-dichloroethane, chloroform, carbon tetrachloride, acetonitrile, acetone, ethyl acetate, methyl acetate, benzene, toluene, mesitylene, xylene, chlorobenzene, ethyl ether, ethylene glycol dimethyl ether, methyl tert-butyl ether, diphenyl ether, 1,4-dioxane, N,N-dimethylformamide, N,N-dimethylacetamide, tetrahydrofuran, 2-methyltetrahydrofuran and a mixture thereof; Adding dehydrating reagent P, which is selected from a group consisting of dicyclohexylcarbodiimide (DCC), polyphosphoric acid, Burgess reagent, bis[a,a-bis(trifluoromethyl)phenethyl alcohol]-diphenylsulfide, carbon disulfide, methyl iodide, sodium hydroxide, potassium hydroxide, lithium hydroxide, sodium hydride, potassium tert-butoxide, sodium tert-butoxide, sodium methoxide, sodium ethoxide, n-butyl lithium, lithium diisopropylamide, cyanuric chloride and a mixture thereof, 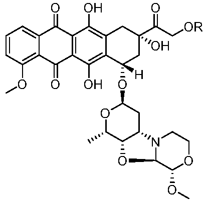 is obtained by reaction, reaction temperature is 78°Csubzero to 25°C, preferably the reaction temperature is 40°C subzero to 25°C. After the reaction is complete, perform a separation operation, preferably by evaporating the solvent under reduced pressure, and then purify or recrystallize by medium pressure chromatography to obtain

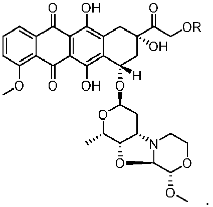

Dissolve the obtained compound 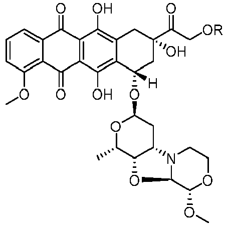 in an appropriate solvent Q, which is selected from a group consisting of dichloromethane, 1,2-dichloroethane, chloroform, carbon tetrachloride, acetonitrile, methanol, ethanol, acetone, ethyl acetate, methyl acetate, benzene, toluene, mesitylene, xylene, chlorobenzene, diethyl ether, ethylene glycol dimethyl ether, methyl tert-butyl ether, diphenyl ether, 1,4-dioxane, N,N-di methylformamide, N,N-dimethylacetamide, tetrahydrofuran, 2-methyltetrahydrofuran and a mixture thereof, add deprotection reagent S, reagent S is selected from a group consisting of hydrochloric acid methanol solution, hydrochloric acid ethanol solution, 1,4-dioxane hydrochloride solution, hydrochloric acid ether solution, tetrahydrofuran hydrochloride, tetrahydrofuran acetate solution, tetramethylammonium fluoride, tetraethylammonium fluoride, tetra-n-butylammonium fluoride (TBAF), Pd/C catalytic hydrogenation, DDQ, p-toluenesulfonic acid, methanol/sodium hydroxide, methanol/sodium methoxide and a mixture thereof. 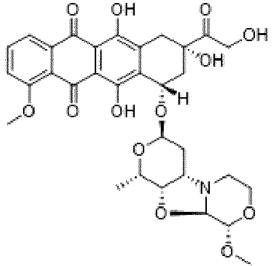 is obtained by deprotection reaction, the reaction temperature is 50°C subzero to 50°C, preferably 30°C subzero to 25°C. After the completion of the reaction, a separation operation is carried out, preferably by evaporating the solvent under reduced pressure, and then purifying or recrystallizing by medium pressure chromatography to obtain PNU-159682

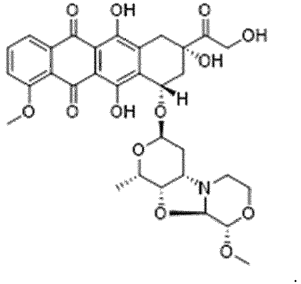

### Example 1

The reaction route is as follows:

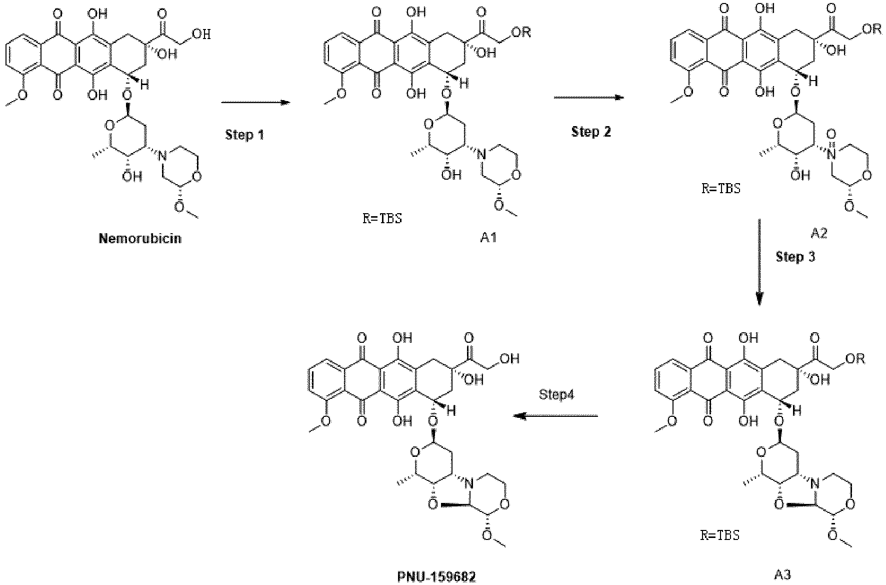

### Step 1:

When R=TBS, the synthesis of A1:
1) Take 20g of 1.0eq of Neumorubicin, 8V/160ml anhydrous DMF, stir at room temperature, and internal temperature of 10-25 degrees.
2) Add 2.5eq/5.28g imidazole, stir for 2min, add TBSC1 (1.5eq)/7.02g in two batches at intervals of 5min and the temperature is not more than 25 degree, and stir at room temperature.
3) Take samples at an interval of 2 hours by HPLC to detect the reaction of the raw materials, and the reaction is complete in about 5-8 hours.
4) Post-treatment after the reaction : Take 5V (relative to DMF) water and add dropwise to the reaction system under mechanical stirring. Use an oil pump to remove water and pull dry or freeze-dry with a lyophilizer to obtain a dark red powdery solid product A1.

### Step 2. Synthesis of A2:

1) Take 10g of A1 (1.0eq), 130ml of 13V anhydrous DCM, stir and lower the internal temperature to 40 subzero to -35 degree.
2) Add m-CPBA (85% 1.02eq) to the reaction system in three batches at 10 min intervals. After 30 minutes of addition, start sampling and detection. HPLC/LCMS detect separately, and the reaction is complete in about 1 hour.
3) HPLC confirms that the reaction is complete, the KI starch test paper detects the remaining oxides in the system, and the test paper does not turn blue, proceed to the next step.

### Step 3. Synthesis of A3

4) Dissolve 3.5eq 1.1g of Burgess reagent in 20ml DCM, add dropwise to the reaction system, remove from the low temperature bath after addition, naturally warm to room temperature 20 to 25 degree and react for 4-8h, check by HPLC/LCMS to confirm that the reaction is complete.
5) Post-treatment: Rotary evaporating the system to remove 2/3 of the total volume of solvent and purified on a chromatographic column.
6) Purification conditions: DCM-10% EtOH/DCM gradient increase.
7) Finally, a dark reddish brown solid product A3 is obtained.

### Step 4:

1) Take A3 1.0eq/16g, 10V /160ml anhydrous THF, stir to dissolve, cool to 3 subzero to 2 degree, add TBAF 1.3eq /27.5ml (1.0M in THF) dropwise to the reaction system, after 5min, start sampling and LCMS/ HPLC-40 detects that the reaction is complete within 30 minutes.
2) Post-treatment: add to the system with 10V/160ml/water, concentrate to remove THF and other solvents. Apply the crude product system directly to the column by wet method, rinse the bottle with a small amount of water, and purify through reversed-phase column under medium pressure.
3) Purification conditions: 0∼20min 10% acetonitrile/ammonium bicarbonate water, 20∼120min 10%∼90% acetonitrile/ammonium bicarbonate water, after all the products come out, rinse the column
4) Mix the product and freeze-dry to obtain 10g product with a yield of 60%.

### Example 2

The reaction route is as follows:

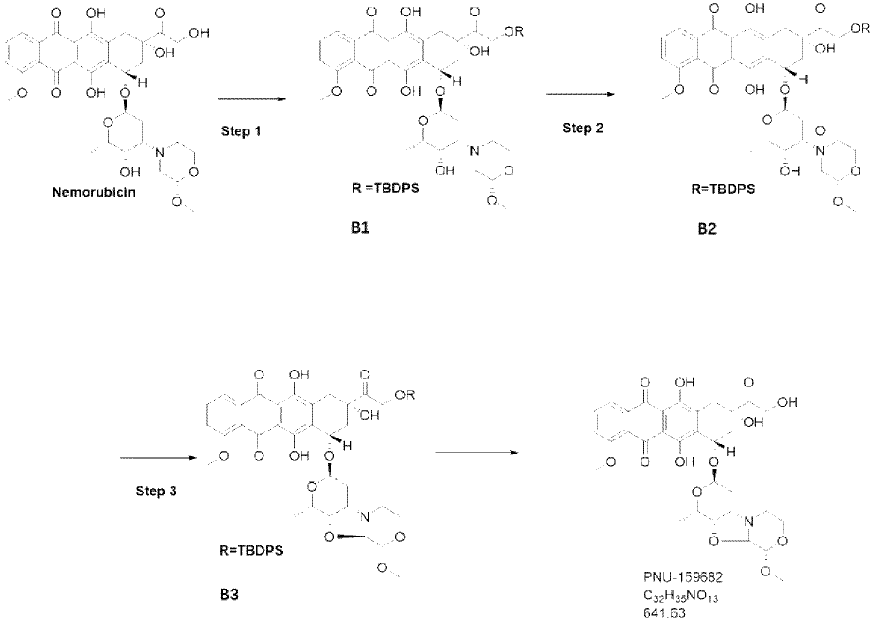

### Step 1:

When R=TBDPS, the synthesis of B1:
1) Nem 1.0 eq 200 mg, 8 V/4 ml anhydrous DMF, stir at room temperature, internal temperature 20 to 25 degree.
2) Add 2.5 eq/53 mg of imidazole, stir for 2 min, add TBDPSCl (1.5 eq)/128 mg to the reaction solution, control the temperature not to exceed 25 degree, and stir at room temperature.
3) Take samples at an interval of 2 hours by HPLC to detect the reaction of the raw materials, and the reaction is complete in about 5-8 hours.
4) Post-treatment after the reaction is complete: Take 10 V (relative to DMF) water and pour the reaction system into the water while stirring at room temperature. Add to the system with ethyl acetate 10 V, (relative to DMF) extraction and liquid separation 3 times, mixmix the organic phases and wash with water 10 V (relative to DMF) 3 times, concentrate and spin-dry, purify by column to obtain a dark red brown solid product B1 (150 mg, 54.7%).

### Step 2. Synthesis of B2:

1) Take B1 2.0g (1.0eq), 13V anhydrous DCM 26ml, stir and reduce the internal temperature 55 degree subzero to 60 degree subzero.
2) Add 0.47g of m-CPBA (85% 1.01eq) to the reaction system in three batches at 10 min intervals. After 30 minutes of addition, start sampling and detection, HPLC/LCMS detect separately. The reaction is complete in about 1 to 2 hours, to obtain B2.
3) KI starch test paper detects the remaining oxides of the system, the test paper does not turn blue, proceed to the next step.

### Step 3. Synthesis of B3:

4) Dissolve 3.5eq 1.89g of Burgess reagent in 20ml DCM, add dropwise to the reaction system, remove from the low temperature bath after adding, naturally warm to room temperature 20 to 25 degree and react for 8-15h, detect by HPLC/LCMS to confirm that the reaction is complete .
5) Post-treatment: Rotary evaporate the system to remove 2/3 of the total volume of solvent and purifyd on a chromatographic column.
6) Purification conditions: DCM-10% EtOH/DCM gradient increase.
7) Finally obtain a deep reddish brown solid product B3.

Step 4, referring to Step 4 of Example 1, PNU-159682 is obtained with a yield of 50%.

### Example 3

The reaction route is as follows:

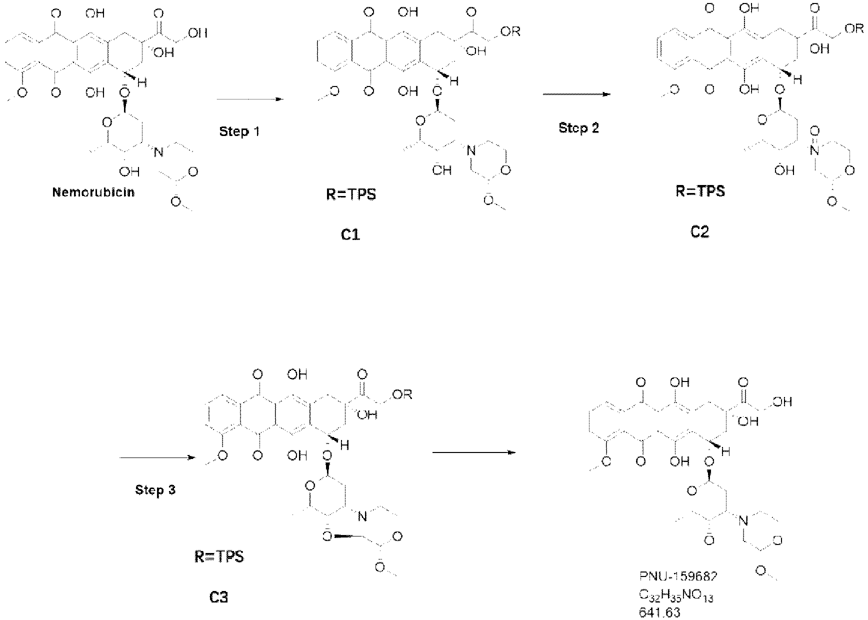

### Step 1:

When R=TPS, the synthesis of C1:
1) Nem 1.0 eq 150 mg, 8 V/4 ml anhydrous DMF, stirat room temperature, internal temperature 20 to 25 degree.
2) Add 3.5 eq/55 mg of imidazole, stir for 2 min, add TPSCl (2.0 eq)/128 mg to the reaction solution, control the temperature not to exceed 25 degrees, and stir at room temperature.
3) Take samples at 2h intervals to detect the reaction of the raw materials by HPLC, and the reaction is basically complete in about 5-8h.
4) Post-treatment after the reaction is complete: Take 10 V (relative to DMF) water and pour the reaction system into the water while stirring at room temperature. Add ethyl acetate 10 V to the system, extract and separate three times (relative to DMF), mix the organic phases and wash 3 times with 10 V (relative to DMF), concentrate and spin dry, purify by column to obtain a dark reddish brown solid product C1 (60 mg, 28.7%).

### Step 2. Synthesis of C2:

1) Take C1 2.0g (1.0eq), 13V anhydrous DCM 26ml, stir and reduce the internal temperature 55 degree subzero to 60 degree subzero.
2) Add 0.46g of m-CPBA (85% 1.01eq) to the reaction system in three batches at 10 min intervals. After 30 minutes of addition, start sampling and detection, HPLC/LCMS detect separately. The reaction is complete in about 1 to 2 hours to obtain C2.
3) KI starch test paper detects the remaining oxides of the system, the test paper does not turn blue, proceed to the next step.

### Step 3. Synthesis of C3:

4) Dissolve 3.5eq 1.85g of Burgess reagent in 20ml DCM, add dropwise to the reaction system, remove from the low temperature bath after adding, naturally warm to room temperature 20 to 25 degree and react for 8-15h, detect by HPLC/LCMS to confirm that the reaction is complete.
5) Post-treatment: Rotary evaporate the system to remove 2/3 of the total volume of solvent and purify on a chromatographic column.
6) Purification conditions: DCM-10% EtOH/DCM gradient increase.
7) Finally, obtain a deep reddish-brown solid product C3.

Step 4, referring to Step 4 of Example 1, obtain PNU-159682 with a yield of 50%.

### Example 4

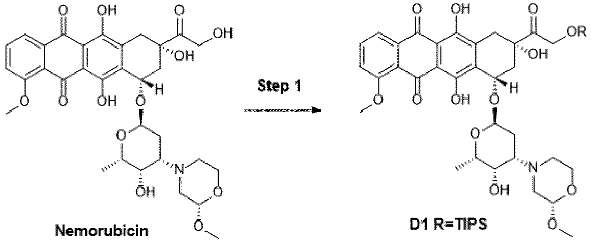

### Step 1:

When R=TIPS, the synthesis of D1:
1) Nem 1.0 eq 150 mg, 8 V/4 ml anhydrous DMF, stir at room temperature, internal temperature 20 to 25 degree.
2) Add 2.5 eq/40 mg imidazole, 0.5 eq/14 mg DMAP, stir for 5 min under ice-water bath conditions, TIPSCl (3.0 eq)/135 mg was added to the reaction solution under ice-water bath conditions, and stir at room temperature.
3) Take samples at 1 hour intervals to detect the reaction of the raw materials by HPLC, and the reaction is complete in about 3 hours.
4) Post-treatment after the reaction is complete: Take a 10 V (relative to DMF) saturated aqueous ammonium chloride solution and pour the reaction system into it under stirring at room temperature. Add ethyl acetate 10 V to the system, (relative to DMF) extraction and separation 3 times, mix the organic phases and wash with water 10 V (relative to DMF) 3 times, concentrate and spin-dry to obtain a dark reddish brown solid product D1 (100 mg, 53.7%).

Steps 2, 3, 4, refer to Example 1, to obtain PNU-159682 with a yield of 25%.

## Claims

1. An intermediate compound for synthesis of PNU-159682, a structural formula of which is: 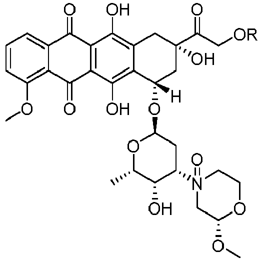 ,wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv.

2. An intermediate compound for synthesis of PNU-159682, a structural formula of which is: 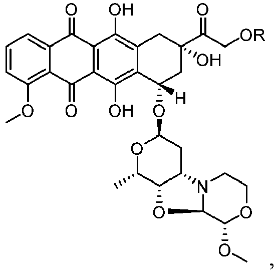 wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, Triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv.

3. A method for synthesizing an intermediate compound 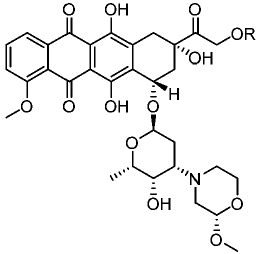 of PNU-159682, wherein , the method is: in solvent X, in the presence of the reagent Y, a compound of structural formula 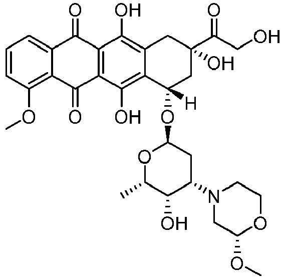 is substituted by a protective group R to obtain an intermediate compound of structural formula: 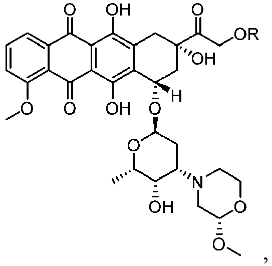
wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv;
Solvent X is selected from a group consisting of dichloromethane, 1,2-dichloroethane, chloroform, carbon tetrachloride, acetonitrile, acetone, ethyl acetate, methyl acetate, water, benzene, ethyl ether, ethylene glycol dimethyl ether, methyl tert-butyl ether, diphenyl ether, 1,4-dioxane, N, N-dimethylformamide, N, N-dimethylacetamide, tetrahydrofuran, 2-methyltetrahydrofuran, and a mixture thereof;
Reagent Y is selected from a group consisting of 4-dimethylaminopyridine, pyridine, imidazole, trimethylamine, triethylamine, diisopropyldiamine and a mixture thereof;
Reaction temperature is 20 °C subzero to 80 °C.

4. The method according to claim 3, wherein the solvent X is N,N-dimethylformamide, the reagent Y is imidazole, and the reaction temperature is 20°C to 25°C.

5. A method for synthesizing an intermediate compound 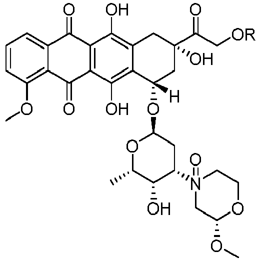 of PNU-159682, wherein, the method is: a compound of structural formula 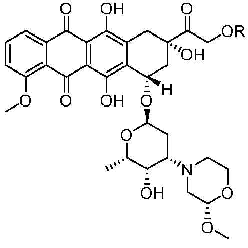 is in solvent M, and add oxidizing reagent N to perform an oxidation reaction to obtain a compound of structural formula 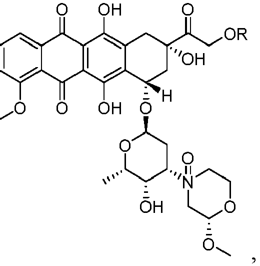
wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv;
The solvent M is selected from a group consisting of dichloromethane, 1,2-dichloroethane, chloroform, carbon tetrachloride, acetonitrile, acetone, ethyl acetate, methyl acetate, water, benzene, ether, ethylene glycol dimethyl ether, methyl tert-butyl ether, diphenyl ether, 1,4-dioxane, N,N-dimethylformamide, N,N-dimethylacetamide, tetrahydrofuran, 2-methyltetrahydrofuran and a mixture thereof;
The reagent N is selected from a group consisting of Jones reagent, Collins reagent (CrO₃.2Py), pyridinium chlorochromate (PCC), pyridine dichromate (PDC), manganese dioxide, DMSO, Dess-Martin oxidant, potassium permanganate, periodic acid, osmium tetroxide, 30% hydrogen peroxide, m-chloroperoxybenzoic acid m-CPBA, tert-butyl hydroperoxide TBHP, acetone peroxy (DMDO) and a mixture thereof;
Reaction temperature is 78°C subzero to 25°C.

6. The method according to claim 5, wherein the reagent N is m-chloroperoxybenzoic acid m-CPBA, the solvent M is methylene chloride, and the reaction temperature is 40°C subzero to 0°C.

7. A method for synthesizing an intermediate compound 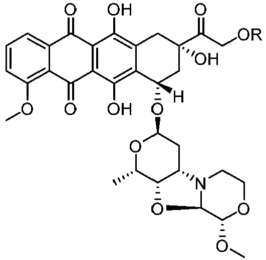 of PNU-159682, wherein, the method is: in solvent O, perform a dehydration cyclization reaction on a compound of structure 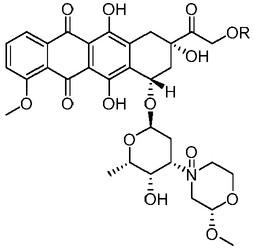 in the presence reagent P to generate an intermediate compound 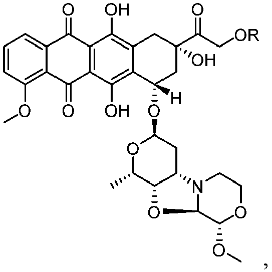
wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, Triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv;
The solvent O is selected from a group consisting of dichloromethane, 1,2-dichloroethane, trichloromethane, carbon tetrachloride, acetonitrile, acetone, ethyl acetate, methyl acetate, benzene, toluene, mesitylene, xylene, chlorobenzene, diethyl ether, ethylene glycol dimethyl ether, methyl tert-butyl ether, diphenyl ether, 1,4-dioxane, N,N-dimethylformamide, N,N-dimethylacetamide, tetrahydrofuran, 2-methyltetrahydrofuran and a mixture thereof;
The solvent O is selected from a group consisting of dichloromethane, 1,2-dichloroethane, chloroform, carbon tetrachloride, acetonitrile, acetone, ethyl acetate, methyl acetate, benzene, toluene, mesitylene, xylene, chlorobenzene, diethyl ether, ethylene glycol dimethyl ether, methyl tert-butyl ether, diphenyl ether, 1,4-dioxane, N,N-dimethylformamide, N,N-dimethylacetamide, tetrahydrofuran, 2-methyltetrahydrofuran, and a mixture thereof;
The reagent P is selected from a group consisting of dicyclohexylcarbodiimide (DCC), polyphosphoric acid, Burgess reagent, bis[a,a-bis(trifluoromethyl)phenethyl alcohol]-diphenylsulfide, carbon disulfide, iodomethane, sodium hydroxide, potassium hydroxide, lithium hydroxide, sodium hydride, potassium tert-butoxide, sodium tert-butoxide, sodium methoxide, sodium ethoxide, n-butyl lithium, lithium diisopropylamide, cyanuric chloride and a mixture thereof;
Reaction temperature is 50°C subzero to 50°C.

8. The method according to claim 7, wherein the reagent P is Burgess reagent, the solvent O is dichloromethane, and the reaction temperature is 40°C subzero to 25°C.

9. A method for synthesizing PNU-159682, wherein, the method is: in solvent Q, perform a deprotection reaction on a compound of structure 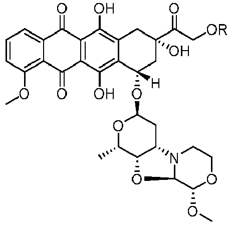 in the presence reagent S, to generate compound PNU-159682 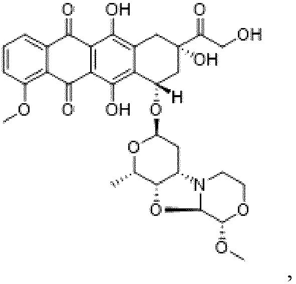
wherein, R is selected from a group consisting of trimethylsilyl TMS, tert-butyldimethylsilyl TBS, tert-butyldiphenylsilyl TBDPS, diethyl isopropylsilyl DEIPS, triisopropylsilyl TIPS, triphenylsilyl TPS, trimethylsilyl Mes, benzyl Bn, p-methoxybenzyl PMB, trityl Tr, 2-tetrahydropyranyl THP, methoxymethyl MOM, 2-ethoxyethyl EE, 2-(trimethylsilyl)ethoxymethyl SEM, Allyl, acetyl Ac, benzoyl Bz, and pivaloyl Pv;
The solvent Q is selected from a group consisting of dichloromethane, 1,2-dichloroethane, chloroform, carbon tetrachloride, acetonitrile, methanol, ethanol, acetone, ethyl acetate, methyl acetate, benzene, toluene, mesitylene, xylene, chlorobenzene, ethyl ether, ethylene glycol dimethyl ether, methyl tert-butyl ether, diphenyl ether, 1,4-dioxane, N,N-dimethylformamide, N,N dimethylacetamide, tetrahydrofuran, 2-methyltetrahydrofuran and a mixture thereof;
The reagent S is selected from a group consisting of hydrochloric acid methanol solution, hydrochloric acid ethanol solution, hydrochloric acid 1,4-dioxane solution, hydrochloric acid ether solution, hydrochloric acid tetrahydrofuran, tetrahydrofuran acetate solution, tetramethylammonium fluoride, tetraethylammonium fluoride, tetra-n-butylammonium fluoride (TBAF), Pd/C catalytic hydrogenation, DDQ, p-toluenesulfonic acid, methanol/sodium hydroxide, methanol/sodium methoxide and a mixture thereof;
Reaction temperature is 50°C subzero to 50°C.

10. The method according to claim 9, wherein the reagent S is tetra-n-butylammonium fluoride (TBAF), the solvent Q is tetrahydrofuran, and the reaction temperature is 30°C subzero to 25°C.
